# EUROPEAN PATENT SPECIFICATION

(11) **EP 3 515 439 B1**
(45) Date of publication and mention of the grant of the patent: **15.12.2021**
(21) Application number: 17771851.7
(22) Date of filing: 18.09.2017
(51) Int. Cl.: A61K 31/439, A61K 9/12, A61K 47/24, A61K 47/10, A61P 11/00

(54) **PHARMACEUTICAL COMPOSITION COMPRISING TIOTROPIUM BROMIDE**
PHARMAZEUTISCHE ZUSAMMENSETZUNG ENTHALTEND TIOTROPIUMBROMIDE
COMPOSITION PHARMACEUTIQUE COMPRENANT DU BROMURE DE TIOTROPIUM

(30) Priority: 19.09.2016 GB 201615912; 02.12.2016 GB 201620513
(43) Date of publication of application: 31.07.2019
(60) Divisional application: 21184484.0
(73) Proprietor: Mexichem Fluor S.A. de C.V., 78395 San Luis Potosi S.L.P. (MX)
(72) Inventor: CORR, Stuart, Warrington Cheshire WA4 5DH (GB); NOAKES, Timothy James, Nr Mold Flintshire CH7 5JF (GB)
(74) Representative: Potter Clarkson
(86) International application number: PCT/GB2017/052763
(87) International publication number: WO 2018/051132

(56) References cited:
- WO-A1-2004/054580
- WO-A2-2014/064410

## Description

The present invention relates to the delivery of drug formulations from a medical device, such as a metered dose inhaler (MDI), using a propellant comprising 1,1-difluoroethane (HFA-152a). More particularly, the present invention relates to pharmaceutical compositions comprising HFA-152a propellant and a drug formulation which is dissolved or suspended in the propellant and to medical devices containing those compositions. The pharmaceutical compositions of the invention are particularly suited for delivery from a pressurised aerosol container using a metered dose inhaler (MDI).

MDIs are the most significant type of inhalation drug delivery system and are well known to those skilled in the art. They are designed to deliver, on demand, a discrete and accurate amount of a drug to the respiratory tract of a patient using a liquefied propellant in which the drug is dissolved, suspended or dispersed. The design and operation of MDIs is described in many standard textbooks and in the patent literature. They all comprise a pressurised container that holds the drug formulation, a nozzle and a valve assembly that is capable of dispensing a controlled quantity of the drug through the nozzle when it is activated. The nozzle and valve assembly are typically located in a housing that is equipped with a mouth piece. The drug formulation will comprise a propellant, in which the drug is dissolved, suspended or dispersed, and may contain other materials such as polar excipients, surfactants and preservatives.

In order for a propellant to function satisfactorily in MDIs, it needs to have a number of properties. These include an appropriate boiling point and vapour pressure so that it can be liquefied in a closed container at room temperature but develop a high enough pressure when the MDI is activated to deliver the drug as an atomised formulation even at low ambient temperatures. Further, the propellant should be of low acute and chronic toxicity and have a high cardiac sensitisation threshold. It should have a high degree of chemical stability in contact with the drug, the container and the metallic and non-metallic components of the MDI device, and have a low propensity to extract low molecular weight substances from any elastomeric materials in the MDI device. The propellant should also be capable of maintaining the drug in a homogeneous solution, in a stable suspension or in a stable dispersion for a sufficient time to permit reproducible delivery of the drug in use. When the drug is in suspension in the propellant, the density of the liquid propellant is desirably similar to that of the solid drug in order to avoid rapid sinking or floating of the drug particles in the liquid. Finally, the propellant should not present a significant flammability risk to the patient in use. In particular, it should form a non-flammable or low flammability mixture when mixed with air in the respiratory tract.

Dichlorodifluoromethane (R-12) possesses a suitable combination of properties and was for many years the most widely used MDI propellant, often blended with trichlorofluoromethane (R-11). Due to international concern that fully and partially halogenated chlorofluorocarbons (CFCs), such as dichlorodifluoromethane and trichlorofluoromethane, were damaging the earth's protective ozone layer, many countries entered into an agreement, the Montreal Protocol, stipulating that their manufacture and use should be severely restricted and eventually phased out completely. Dichlorodifluoromethane and trichlorofluoromethane were phased out for refrigeration use in the 1990's, but are still used in small quantities in the MDI sector as a result of an essential use exemption in the Montreal Protocol.

1,1,1,2-tetrafluoroethane (HFA-134a) was introduced as a replacement refrigerant and MDI propellant for R-12. 1,1,1,2,3,3,3-heptafluoropropane (HFA-227ea) was also introduced as a replacement propellant for dichlorotetrafluoroethane (R-114) in the MDI sector and is sometimes used alone or blended with HFA -134a for this application.

Although HFA-134a and HFA-227ea have low ozone depletion potentials (ODPs), they have global warming potentials (GWPs), 1430 and 3220 respectively, which are now considered to be too high by some regulatory bodies, especially for dispersive uses when they are released into the atmosphere.

One industrial area that has received particular attention recently has been the automotive air-conditioning sector where the use of HFA-134a has come under regulatory control as a result of the European Mobile Air Conditioning Directive (2006/40/EC). Industry is developing a number of possible alternatives to HFA-134a in automotive air conditioning and other applications that have a low greenhouse warming potential (GWP) as well as a low ozone depletion potential (ODP). Many of these alternatives include hydrofluoropropenes, especially the tetrafluoropropenes, such as 2,3,3,3-tetrafluoropropene (HFO-1234yf) and 1,3,3,3-tetrafluoropropene (HFO-1234ze).

Although the proposed alternatives to HFA-134a have a low GWP, the toxicological status of many of the components, such as certain of the fluoropropenes, is unclear and they are unlikely to be acceptable for use in the MDI sector for many years, if at all.

Tiotropium bromide ((1α, 2β, 4β, 5α, 7β)-7-[(hydroxydi-2-thienylacetyl)oxy]-9,9-dimethyl-3-oxa-9-azoniatricyclo[3.3.1.0^{2,4}]nonane bromide)), particularly in the form of its monohydrate, is a long-acting muscarinic anticholinergic (LAMA) bronchodilator used in the management of chronic obstructive pulmonary disease (COPD).

Unfortunately, it has proven difficult to formulate tiotropium in a form that is suitable for delivery using a MDI due to its limited physical and chemical stability. The problem of stability may be particularly evident when the tiotropium is exposed to other components that are often used in pharmaceutical formulations, including excipients, solvents, e.g. ethanol, and other therapeutic agents.

The instability of pharmaceutical formulations of tiotropium can result in a limited shelf life at ambient temperatures and can necessitate refrigerated storage prior to use.

US2003/171586 describes the manufacture of crystalline tiotropium bromide as its monohydrate and notes that it can be propelled in aerosol form using HFA-134a or HFA-227ea. US2003/171586 also highlights the importance of chemical stability in determining the shelf life and safety of medicaments and that any improvement in physical or chemical stability of tiotropium formulations is an important advantage.

There is a need for a pharmaceutical composition of tiotropium which can be delivered using a MDI and that uses a propellant having a reduced GWP in comparison with HFA-134a and HFA-227ea. There is also a need for a pharmaceutical composition of tiotropium which exhibits improved stability.

We have found that the issues associated with the use of tiotropium-based formulations in MDIs may be overcome by using a propellant that comprises 1,1-difluoroethane (HFA-152a), particularly where the formulations contain low amounts of water. These formulations can exhibit improved chemical stability, improved aerosolisation performance for improved drug delivery, good suspension stability, reduced GWP, good compatibility with standard uncoated aluminium cans as well as good compatibility with standard valves and seals.

According to a first aspect of the present invention, there is provided a pharmaceutical composition as claimed in claim 1 and as claimed in claim 2.

The pharmaceutical composition of the first aspect of the invention typically contains less than 500 ppm of water based on the total weight of the pharmaceutical composition. The improved chemical stability is observed, in particular, when the pharmaceutical composition contains less than 100 ppm, preferably less than 50 ppm, more preferably less than 10 ppm and particularly less than 5 ppm of water based on the total weight of the pharmaceutical composition. In referring to the water content of the pharmaceutical composition, we are referring to the content of free water in the composition and not any water that happens to be present in any hydrated drug compounds that may be used as part of the drug component. In an especially preferred embodiment, the pharmaceutical composition is water-free. Alternatively, the pharmaceutical composition of the first aspect may contain greater than 0.5 ppm of water, e.g. greater than 1 ppm, but less than the amounts discussed above, as it can in practice be difficult to remove all the water from the composition and then retain it in such a water-free state.

Accordingly a preferred embodiment of the first aspect of the present invention provides a pharmaceutical composition as claimed in claim 1 or claim 2, wherein the composition contains less than 100 ppm, preferably less than 50 ppm, more preferably less than 10 ppm and especially less than 5 ppm of water based on the total weight of the pharmaceutical composition.

In a preferred embodiment, the pharmaceutical composition of the first aspect of the invention contains less than 1000 ppm, preferably less than 500 ppm, more preferably less than 100 ppm and particularly less than 50 ppm of dissolved oxygen based on the total weight of the pharmaceutical composition. In an especially preferred embodiment, the pharmaceutical composition is oxygen-free. Alternatively, the pharmaceutical composition of the first aspect may contain greater than 0.5 ppm of oxygen, e.g. 1 ppm or greater, but less than the amounts discussed above, as it can in practice be difficult to retain the composition in an oxygen-free state. Low oxygen contents are preferred because they tend to reduce the degradation of the drug compounds resulting in a composition with higher chemical stability.

Accordingly, a preferred embodiment of the first aspect of the present invention provides a pharmaceutical composition as claimed in claim 1 or claim 2, wherein the composition contains less than 1000 ppm, preferably less than 500 ppm, more preferably less than 100 ppm and especially less than 50 ppm of oxygen based on the total weight of the pharmaceutical composition.

The pharmaceutical composition of the present invention is suitable for delivery to the respiratory tract using a metered dose inhaler (MDI).

The tiotropium bromide monohydrate in the pharmaceutical composition of the invention in all aspects and embodiments disclosed herein is preferably in a micronized form. Further, the pharmaceutical composition of the invention in all aspects and embodiments disclosed herein is preferably free of perforated microstructures.

The tiotropium bromide monohydrate may be dispersed or suspended in the propellant. The drug particles in such suspensions preferably have a diameter of less than 100 microns, e.g. less than 50 microns. However, in an alternative embodiment the pharmaceutical compositions of the invention are solutions with the tiotropium bromide monohydrate dissolved in the propellant, e.g. with the assistance of a polar excipient, such as ethanol.

The amount of the drug component in the pharmaceutical composition of the first aspect of the present invention will typically be in the range of from 0.01 to 2.5 weight % based on the total weight of the pharmaceutical composition. Preferably, the drug component will comprise from 0.01 to 2.0 weight %, more preferably from 0.05 to 2.0 weight % and especially from 0.05 to 1.5 weight % of the total weight of the pharmaceutical composition. The drug component may consist essentially of or consist entirely of the tiotropium bromide monohydrate. By the term "consists essentially of", we mean that at least 98 weight %, more preferably at least 99 weight % and especially at least 99.9 weight % of the drug component consists of the tiotropium bromide monohydrate. Alternatively, the drug component may contain other drugs, such as at least one long acting beta-2 agonist (LABA) and/or at least one corticosteroid.

The propellant component in the pharmaceutical composition of the first aspect of the present invention comprises 1,1-difluoroethane (HFA-152a). Thus, we do not exclude the possibility that the propellant component may include other propellant compounds in addition to the HFA-152a. For example, the propellant component may additionally comprise one or more additional hydrofluorocarbon or hydrocarbon propellant compounds, e.g. selected from HFA-227ea, HFA-134a, difluoromethane (HFA-32), propane, butane, isobutane and dimethyl ether. The preferred additional propellants are HFA-227ea and HFA-134a.

If an additional propellant compound is included, such as HFA-134a or HFA-227ea, at least 90 weight %, e.g. from 90 to 99 weight %, of the propellant component should be HFA-152a. Preferably, the HFA-152a will constitute at least 95 weight %, e.g. from 95 to 99 weight %, and more preferably at least 99 weight % of the propellant component.

In a preferred embodiment, the propellant component has a global warming potential (GWP) of less than 250, more preferably less than 200 and still more preferably less than 150.

In an especially preferred embodiment, the propellant component consists entirely of HFA-152a so that the pharmaceutical composition of the invention comprises HFA-152a as the sole propellant. By the term "consists entirely of" we do not, of course, exclude the presence of minor amounts, e.g. up to a few hundred parts per million, of impurities that may be present following the process that is used to make the HFA-152a providing that they do not affect the suitability of the propellant in medical applications. Preferably the HFA-152a propellant will contain no more than 10 ppm, e.g. from 0.5 to 10 ppm, more preferably no more than 5 ppm, e.g. from 1 to 5 ppm, of unsaturated impurities, such as vinyl fluoride, vinyl chloride, vinylidene fluoride and chloro-fluoro ethylene compounds.

The amount of propellant component in the pharmaceutical composition of the invention will vary depending on the amounts of the drugs and other components in the pharmaceutical composition. Typically, the propellant component will comprise from 80.0 to 99.99 weight % of the total weight of the pharmaceutical composition. Preferably, the propellant component will comprise from 90.0 to 99.99 weight %, more preferably from 96.5 to 99.99 weight % and especially from 97.5 to 99.95 weight % of the total weight of the pharmaceutical composition.

In one embodiment, the pharmaceutical composition of the first aspect of the present invention consists essentially of and more preferably consists entirely of the drug component and the propellant component. By the term "consists essentially of", we mean that at least 98 weight %, more preferably at least 99 weight % and especially at least 99.9 weight % of the pharmaceutical composition consists of the drug component and the propellant component.

In another embodiment, the pharmaceutical composition of the first aspect of the present invention additionally includes a polar excipient, such as ethanol. Polar excipients have been used previously in pharmaceutical compositions for treating respiratory disorders that are delivered using metered dose inhalers (MDIs). They are also referred to as solvents, co-solvents, carrier solvents and adjuvants. Their inclusion can serve to solubilise the surfactant or the drug in the propellant and/or inhibit deposition of drug particles on the surfaces of the metered dose inhaler that are contacted by the pharmaceutical composition as it passes from the container in which it is stored to the nozzle outlet. They are also used as bulking agents in two-stage filling processes where the drug is mixed with a suitable polar excipient. The most commonly used polar excipient is ethanol. If a polar excipient is used, it will typically be present in an amount of from 0.5 to 10 % by weight, preferably in an amount of from 1 to 5 % by weight based on the total weight of the pharmaceutical composition.

In one preferred embodiment, the pharmaceutical composition of the present invention is free of polar excipients such as ethanol.

The pharmaceutical composition of the first aspect of the present invention may also include a surfactant component comprising at least one surfactant compound. Surfactant compounds of the type that have been in use hitherto in pharmaceutical formulations for MDIs may be used in the pharmaceutical compositions of the present invention. Preferred surfactants are selected from polyvinylpyrrolidone, polyethylene glycol surfactants, oleic acid and lecithin. By the term oleic acid, we are not necessarily referring to pure (9Z)-octadec-9-enoic acid. When sold for surfactant use in medical applications, oleic acid is typically a mixture of several fatty acids, with (9Z)-octadec-9-enoic acid being the predominant fatty acid, e.g. present in an amount of at least 65 weight % based on the total weight of the surfactant.

In a preferred embodiment, the surfactant component consists essentially of and still more preferably consists entirely of at least one surfactant compound selected from polyvinylpyrrolidone, polyethylene glycols, oleic acid and lecithin. In a particularly preferred embodiment, the surfactant component consists essentially of and still more preferably consists entirely of at least one surfactant compound selected from polyvinylpyrrolidone and polyethylene glycols. By the term "consists essentially of", we mean that at least 95 weight %, more preferably at least 98 weight % and especially at least 99 weight % of the surfactant component is composed of the listed surfactants.

If a surfactant component is used, it will typically be present in an amount of from 0.1 to 2.5 % by weight, preferably in an amount of from 0.2 to 1.5 % by weight based on the total weight of the pharmaceutical composition.

The pharmaceutical composition of the invention may also include a long acting beta-2-agonist (LABA). Any of the long acting beta-2-agonists that have been in use hitherto for treating asthma and chronic obstructive pulmonary diseases and that can be delivered using a MDI can be used in the pharmaceutical compositions of the present invention. Suitable long acting beta-2-agonists include formoterol, arformoterol, bambuterol, clenbuterol, salmeterol, indacaterol, olodaterol and vilanterol as well as their pharmaceutically acceptable derivatives, such as their pharmaceutically acceptable salts. Preferred compounds include formoterol, salmeterol and olodaterol and the pharmaceutically acceptable salts thereof.

Particularly preferred compounds include formoterol fumarate, formoterol fumarate dihydrate, salmeterol xinafoate and oladaterol.

Accordingly, a second aspect of the present invention provides a pharmaceutical composition, e.g. a pharmaceutical suspension or a pharmaceutical solution, said composition comprising:
(i) a drug component comprising tiotropium bromide monohydrate, and at least one long acting beta-2-agonist (LABA), especially at least one long acting beta-2 agonist (LABA) selected from formoterol, salmeterol and olodaterol and the pharmaceutically acceptable salts thereof; and
(ii) a propellant component comprising 1,1-difluoroethane (HFA-152a).

When the composition of the second aspect of the present invention is a solution it is free of acid stabilisers.

The pharmaceutical composition of the second aspect of the invention typically contains less than 500 ppm of water based on the total weight of the pharmaceutical composition. Preferably, the pharmaceutical composition of the second aspect of the present invention contains less than 100 ppm, more preferably less than 50 ppm, particularly less than 10 ppm and especially less than 5 ppm of water based on the total weight of the pharmaceutical composition. It has been found that small amounts of water alongside the use of HFA-152a as the propellant can result in a pharmaceutical composition with improved chemical stability. In referring to the water content of the pharmaceutical composition, we are referring to the content of free water in the composition and not any water that happens to be present in any hydrated drug compounds that may be used as part of the drug component. In an especially preferred embodiment, the pharmaceutical composition of the second aspect of the present invention is water-free. Alternatively, the pharmaceutical composition of the second aspect may contain greater than 0.5 ppm of water, e.g. greater than 1 ppm, but less than the amounts discussed above, as it can in practice be difficult to remove all the water from the composition and then retain it in such a water-free state.

In a preferred embodiment, the pharmaceutical composition of the second aspect of the invention contains less than 1000 ppm, preferably less than 500 ppm, more preferably less than 100 ppm and particularly less than 50 ppm of dissolved oxygen based on the total weight of the pharmaceutical composition. In an especially preferred embodiment, the pharmaceutical composition is oxygen-free. Alternatively, the pharmaceutical composition of the second aspect may contain greater than 0.5 ppm of oxygen, e.g. 1 ppm or greater, but less than the amounts discussed above, as it can in practice be difficult to retain the composition in an oxygen-free state. Low oxygen contents are preferred because they tend to reduce the degradation of the drug compounds resulting in a composition with higher chemical stability.

Typical and preferred amounts of the drug component and the propellant component in the pharmaceutical composition of the second aspect of the present invention and suitable, typical and preferred compositions for the propellant component are as discussed above for the pharmaceutical composition of the first aspect of the invention. The drug component may consist essentially of or consist entirely of the tiotropium bromide monohydrate and the at least one long acting beta-2 agonist (LABA). By the term "consists essentially of", we mean that at least 98 weight %, more preferably at least 99 weight % and especially at least 99.9 weight % of the drug component consists of the tiotropium bromide monohydrate and the at least one long acting beta-2 agonist (LABA).

In one embodiment, the pharmaceutical composition of the second aspect of the present invention consists essentially of and more preferably consists entirely of the two components (i) and (ii) listed above. By the term "consists essentially of", we mean that at least 98 weight %, more preferably at least 99 weight % and especially at least 99.9 weight % of the pharmaceutical composition consists of the two listed components.

In another embodiment, the pharmaceutical composition of the second aspect of the invention may contain one or both of a polar excipient and a surfactant component as discussed above for the pharmaceutical composition of the first aspect of the invention. Suitable and preferred polar excipients and surfactants are as discussed above for the pharmaceutical composition of the first aspect of the invention. Typical and preferred amounts of the polar excipient and the surfactant component are as discussed above for the pharmaceutical composition of the first aspect of the invention.

In an especially preferred embodiment of the second aspect of the invention, the drug component comprises tiotropium bromide monohydrate, and at least one long acting beta-2-agonist selected from formoterol, salmeterol and olodaterol and the pharmaceutically acceptable salts thereof. Preferably, the tiotropium bromide monohydrate and the at least one selected long acting beta-2-agonist are the only pharmaceutical actives in the pharmaceutical composition of the second aspect of the invention.

The pharmaceutical composition of the invention may also include a corticosteroid. Any of the corticosteroids that have been in use hitherto for treating asthma and chronic obstructive pulmonary diseases and that can be delivered using a MDI can be used in the pharmaceutical compositions of the present invention. Suitable corticosteroids include budesonide, mometasone, beclomethasone and fluticasone as well as their pharmaceutically acceptable derivatives, such as their pharmaceutically acceptable salts and esters. Preferred compounds include budesonide, mometasone furoate, beclomethasone dipropionate and fluticasone propionate. The most preferred corticosteroids are budesonide, mometasone, fluticasone and beclomethasone, particularly budesonide and mometasone and especially budesonide.

Accordingly, a third aspect of the present invention provides a pharmaceutical composition, e.g. a pharmaceutical suspension or a pharmaceutical solution, said composition comprising:
(i) a drug component comprising tiotropium bromide monohydrate, and at least one corticosteroid, particularly at least one corticosteroid selected from fluticasone, budesonide, mometasone and beclomethasone and the pharmaceutically acceptable derivatives thereof, especially budesonide; and
(ii) a propellant component comprising 1,1-difluoroethane (HFA-152a).

When the composition of the third aspect of the present invention is a solution it is free of acid stabilisers.

The pharmaceutical composition of the third aspect of the invention typically contains less than 500 ppm of water based on the total weight of the pharmaceutical composition. Preferably, the pharmaceutical composition of the third aspect of the present invention contains less than 100 ppm, more preferably less than 50 ppm, particularly less than 10 ppm and especially less than 5 ppm of water based on the total weight of the pharmaceutical composition. It has been found that small amounts of water alongside the use of HFA-152a as the propellant can result in a pharmaceutical composition with improved chemical stability. In referring to the water content of the pharmaceutical composition, we are referring to the content of free water in the composition and not any water that happens to be present in any hydrated drug compounds that may be used as part of the drug component. In an especially preferred embodiment, the pharmaceutical composition of the third aspect of the present invention is water-free. Alternatively, the pharmaceutical composition of the third aspect may contain greater than 0.5 ppm of water, e.g. greater than 1 ppm, but less than the amounts discussed above, as it can in practice be difficult to remove all the water from the composition and then retain it in such a water-free state.

In a preferred embodiment, the pharmaceutical composition of the third aspect of the invention contains less than 1000 ppm, preferably less than 500 ppm, more preferably less than 100 ppm and particularly less than 50 ppm of dissolved oxygen based on the total weight of the pharmaceutical composition. In an especially preferred embodiment, the pharmaceutical composition is oxygen-free. Alternatively, the pharmaceutical composition of the third aspect may contain greater than 0.5 ppm of oxygen, e.g. 1 ppm or greater, but less than the amounts discussed above, as it can in practice be difficult to retain the composition in an oxygen-free state. Low oxygen contents are preferred because they tend to reduce the degradation of the drug compounds resulting in a composition with higher chemical stability.

Typical and preferred amounts of the drug component and the propellant component in the pharmaceutical composition of the third aspect of the present invention and suitable, typical and preferred compositions for the propellant component are as discussed above for the pharmaceutical composition of the first aspect of the invention. The drug component may consist essentially of or consist entirely of the tiotropium bromide monohydrate and the at least one corticosteroid. By the term "consists essentially of", we mean that at least 98 weight %, more preferably at least 99 weight % and especially at least 99.9 weight % of the drug component consists of the tiotropium bromide monohydrate and the at least one corticosteroid.

In one embodiment, the pharmaceutical composition of the third aspect of the present invention consists essentially of and more preferably consists entirely of the two components (i) and (ii) listed above. By the term "consists essentially of", we mean that at least 98 weight %, more preferably at least 99 weight % and especially at least 99.9 weight % of the pharmaceutical composition consists of the two listed components.

In another embodiment, the pharmaceutical composition of the third aspect of the invention may contain one or both of a polar excipient and a surfactant component as discussed above for the pharmaceutical composition of the first aspect of the invention. Suitable and preferred polar excipients and surfactants are as discussed above for the pharmaceutical composition of the first aspect of the invention. Typical and preferred amounts of the polar excipient and the surfactant component are as discussed above for the pharmaceutical composition of the first aspect of the invention.

In an especially preferred embodiment of the third aspect of the invention, the drug component comprises tiotropium bromide monohydrate, and budesonide. Preferably, the tiotropium bromide monohydrate and budesonide are the only pharmaceutical actives in the pharmaceutical composition of the third aspect of the invention.

The pharmaceutical composition of the invention may also include a long acting beta-2-agonist (LABA) and a corticosteroid. Any of the long acting beta-2-agonists and corticosteroids that have been in use hitherto for treating asthma and chronic obstructive pulmonary diseases and that can be delivered using a MDI can be used in the pharmaceutical compositions of the present invention. Suitable and preferred long acting beta-2-agonists are as discussed above for the second aspect of the invention. Suitable and preferred corticosteroids are as discussed above for the third aspect of the present invention.

Accordingly, a fourth aspect of the present invention provides a pharmaceutical composition, e.g. a pharmaceutical suspension or a pharmaceutical solution, said composition comprising:
(i) a drug component comprising tiotropium bromide monohydrate, at least one long acting beta-2-agonist (LABA), especially at least one long acting beta-2 agonist (LABA) selected from formoterol, salmeterol and olodaterol and the pharmaceutically acceptable salts thereof and at least one corticosteroid, particularly at least one corticosteroid selected from fluticasone, budesonide, mometasone and beclomethasone and the pharmaceutically acceptable derivatives thereof, especially budesonide; and
(ii) a propellant component comprising 1,1-difluoroethane (HFA-152a).

When the composition of the fourth aspect of the present invention is a solution it is free of acid stabilisers.

The pharmaceutical composition of the fourth aspect of the invention typically contains less than 500 ppm of water based on the total weight of the pharmaceutical composition. Preferably, the pharmaceutical composition of the fourth aspect of the present invention contains less than 100 ppm, more preferably less than 50 ppm, particularly less than 10 ppm and especially less than 5 ppm of water based on the total weight of the pharmaceutical composition. It has been found that small amounts of water alongside the use of HFA-152a as the propellant can result in a pharmaceutical composition with improved chemical stability. In referring to the water content of the pharmaceutical composition, we are referring to the content of free water in the composition and not any water that happens to be present in any hydrated drug compounds that may be used as part of the drug component. In an especially preferred embodiment, the pharmaceutical composition of the fourth aspect of the present invention is water-free. Alternatively, the pharmaceutical composition of the fourth aspect may contain greater than 0.5 ppm of water, e.g. greater than 1 ppm, but less than the amounts discussed above, as it can in practice be difficult to remove all the water from the composition and then retain it in such a water-free state.

In a preferred embodiment, the pharmaceutical composition of the fourth aspect of the invention contains less than 1000 ppm, preferably less than 500 ppm, more preferably less than 100 ppm and particularly less than 50 ppm of dissolved oxygen based on the total weight of the pharmaceutical composition. In an especially preferred embodiment, the pharmaceutical composition is oxygen-free. Alternatively, the pharmaceutical composition of the fourth aspect may contain greater than 0.5 ppm of oxygen, e.g. 1 ppm or greater, but less than the amounts discussed above, as it can in practice be difficult to retain the composition in an oxygen-free state. Low oxygen contents are preferred because they tend to reduce the degradation of the drug compounds resulting in a composition with higher chemical stability.

Typical and preferred amounts of the drug component and the propellant component in the pharmaceutical composition of the fourth aspect of the present invention and suitable, typical and preferred compositions for the propellant component are as discussed above for the pharmaceutical composition of the first aspect of the invention. The drug component may consist essentially of or consist entirely of the tiotropium bromide monohydrate, the at least one long acting beta-2 agonist (LABA) and the at least one corticosteroid. By the term "consists essentially of", we mean that at least 98 weight %, more preferably at least 99 weight % and especially at least 99.9 weight % of the drug component consists of the tiotropium bromide monohydrate, the at least one long acting beta-2 agonist (LABA) and the at least one corticosteroid.

In one embodiment, the pharmaceutical composition of the fourth aspect of the present invention consists essentially of and more preferably consists entirely of the two components (i) and (ii) listed above. By the term "consists essentially of", we mean that at least 98 weight %, more preferably at least 99 weight % and especially at least 99.9 weight % of the pharmaceutical composition consists of the two listed components.

In another embodiment, the pharmaceutical composition of the fourth aspect of the invention may contain one or both of a polar excipient and a surfactant component as discussed above for the pharmaceutical composition of the first aspect of the invention. Suitable and preferred polar excipients and surfactants are as discussed above for the pharmaceutical composition of the first aspect of the invention. Typical and preferred amounts of the polar excipient and the surfactant component are as discussed above for the pharmaceutical composition of the first aspect of the invention.

In an especially preferred embodiment of the fourth aspect of the invention, the drug component comprises tiotropium bromide monohydrate, at least one long acting beta-2-agonist selected from formoterol, salmeterol and olodaterol and the pharmaceutically acceptable salts thereof and budesonide. Preferably, the tiotropium bromide monohydrate, the at least one selected long acting beta-2-agonist and the budesonide are the only pharmaceutical actives in the pharmaceutical composition of the fourth aspect of the invention.

It has been found that the use of propellants comprising 1,1-difluoroethane (HFA-152a) in pharmaceutical compositions containing a tiotropium compound, such as tiotropium bromide monohydrate, and the propellant can unexpectedly improve the chemical stability of the tiotropium compound compared to the stability it exhibits in formulations containing either HFA-134a or HFA-227ea as the propellant.

Accordingly, also described herein is a method of improving the stability of a pharmaceutical composition comprising a propellant component and a drug component comprising at least one tiotropium compound selected from tiotropium and the pharmaceutically acceptable derivatives thereof, said method comprising using a propellant component comprising 1,1-difluoroethane (HFA-152a).

The pharmaceutical composition in the stabilisation method may be a suspension or a solution.

The improved chemical stability can result, in particular, when the pharmaceutical composition contains less than 500 ppm, preferably less than 100 ppm, more preferably less than 50 ppm, still more preferably less than 10 ppm and particularly less than 5 ppm of water based on the total weight of the pharmaceutical composition. In referring to the water content of the pharmaceutical composition, we are referring to the content of free water in the composition and not any water that happens to be present in any hydrated drug compounds that may be used as part of the drug component. In an especially preferred stabilisation method, the pharmaceutical composition is water-free. Alternatively, the pharmaceutical composition that is utilised in the described stabilisation method may contain greater than 0.5 ppm of water, e.g. greater than 1 ppm, but less than the amounts discussed above, as it can in practice be difficult to remove all the water from the composition and then retain it in such a water-free state.

Accordingly, also described herein is a method of improving the stability of a pharmaceutical composition comprising a propellant component and a drug component comprising at least one tiotropium compound selected from tiotropium and the pharmaceutically acceptable derivatives thereof, said method comprising using a propellant component comprising 1,1-difluoroethane (HFA-152a) and selecting the components and conditions for the preparation of the pharmaceutical composition to maintain the water content of the pharmaceutical composition below 100 ppm, preferably below 50 ppm, more preferably below 10 ppm and particularly below 5 ppm based on the total weight of the pharmaceutical composition.

In practice, preparing a pharmaceutical composition with the low water levels recited above involves using a propellant component with a suitably low water content, as it is usually the largest mass item in the finished device, and then preparing the pharmaceutical composition under suitably dry conditions, e.g. in a dry nitrogen atmosphere. Preparing pharmaceutical compositions under dry conditions is well known and the techniques involved are well understood by those skilled in the art. Other steps to obtain a low water content in the finished device include drying and storing the can and valve components in a moisture-controlled atmosphere, e.g. dry nitrogen or air, prior to and during device assembly. If the pharmaceutical composition contains a significant amount of ethanol, then it may also be important to control the water content of the ethanol as well as the propellant, e.g. by drying to reduce the water content to suitably low levels. Suitable drying techniques are well known to those skilled in the art and include the use of a molecular sieve or other inorganic desiccant and membrane drying processes.

In the described stabilisation method suitable pharmaceutically acceptable derivatives of tiotropium include, inter alia, pharmaceutically acceptable salts, pharmaceutically acceptable prodrugs, pharmaceutically acceptable solvates, pharmaceutically acceptable hydrates, pharmaceutically acceptable esters, solvates of pharmaceutically acceptable salts, solvates of pharmaceutically acceptable prodrugs, hydrates of pharmaceutically acceptable salts and hydrates of pharmaceutically acceptable prodrugs. A preferred pharmaceutically acceptable derivative for use in the described stabilisation method is tiotropium bromide, preferably tiotropium bromide monohydrate. In a particularly preferred stabilisation method, the at least one tiotropium compound in the pharmaceutical composition is tiotropium bromide and/or tiotropium bromide monohydrate and more preferably is tiotropium bromide monohydrate.

Typical and preferred amounts of the drug component and the propellant component in the stabilisation method described herein and suitable, typical and preferred compositions for the propellant component are as discussed above for the pharmaceutical composition of the first aspect of the invention.

The drug component in the described stabilisation method may consist essentially of or consist entirely of the at least one tiotropium compound selected from tiotropium and the pharmaceutically acceptable derivatives thereof. By the term "consists essentially of", we mean that at least 98 weight %, more preferably at least 99 weight % and especially at least 99.9 weight % of the drug component consists of the least one tiotropium compound. Alternatively, the drug component may additionally comprise at least one corticosteroid and/or at least one long acting beta-2-agonist. When a corticosteroid and/or a long acting beta-2-agonist are included, suitable and preferred corticosteroids and suitable and preferred long acting beta-2-agonists are as described above for the pharmaceutical compositions of the second and third aspects of the present invention.

In one stabilisation method, the pharmaceutical composition consists essentially of and more preferably consists entirely of the drug component and the propellant component as defined above. By the term "consists essentially of", we mean that at least 98 weight %, more preferably at least 99 weight % and especially at least 99.9 weight % of the pharmaceutical composition consists of the two components.

In an alternative stabilisation method, the pharmaceutical composition may contain one or both of a polar excipient and a surfactant component as discussed above for the pharmaceutical composition of the first aspect of the invention. Suitable and preferred polar excipients and surfactants are as discussed above for the pharmaceutical composition of the first aspect of the invention. Typical and preferred amounts of the polar excipient and the surfactant component are as discussed above for the pharmaceutical composition of the first aspect of the invention.

In one preferred stabilisation method, the resulting pharmaceutical composition after storage at 40°C and 75 % relative humidity for 1 month will produce less than 0.2 % by weight, preferably less than 0.1 % by weight and more preferably less than 0.05 % by weight of impurities from the degradation of the at least one tiotropium compound based on the total weight of the at least one tiotropium compound and the impurities.

In another preferred stabilisation method in which the pharmaceutical composition also comprises at least one corticosteroid and/or at least one long acting beta-2-agonist, the resulting pharmaceutical composition after storage at 40°C and 75 % relative humidity for 1 month will produce less than 0.2 % by weight, preferably less than 0.1 % by weight and more preferably less than 0.05 % by weight of impurities from the degradation of the at least one tiotropium compound based on the total weight of the at least one tiotropium compound and the impurities.

In a further preferred stabilisation method, the resulting pharmaceutical composition after storage at 40°C and 75 % relative humidity for 3 months will produce less than 0.3 % by weight, preferably less than 0.2 % by weight and more preferably less than 0.15 % by weight of impurities from the degradation of the at least one tiotropium compound based on the total weight of the at least one tiotropium compound and the impurities.

In another preferred stabilisation method in which the pharmaceutical composition also comprises at least one corticosteroid and/or at least one long acting beta-2-agonist, the resulting pharmaceutical composition after storage at 40°C and 75 % relative humidity for 3 months will produce less than 0.3 % by weight, preferably less than 0.2 % by weight and more preferably less than 0.15 % by weight of impurities from the degradation of the at least one tiotropium compound based on the total weight of the at least one tiotropium compound and the impurities.

In yet another preferred stabilisation method, at least 97.0 % by weight, preferably at least 98.0 % by weight and more preferably at least 98.5 % by weight of the at least one tiotropium compound that is contained originally in the pharmaceutical composition immediately following preparation will be present in the composition after storage at 40°C and 75 % relative humidity for 3 months.

In still another preferred stabilisation method in which the pharmaceutical composition also comprises at least one corticosteroid and/or at least one long acting beta-2-agonist, at least 97.0 % by weight, preferably at least 98.0 % by weight and more preferably at least 98.5 % by weight of the at least one tiotropium compound that is contained originally in the pharmaceutical composition immediately following preparation will be present in the composition after storage at 40°C and 75 % relative humidity for 3 months.

In a further preferred stabilisation method, at least 97.0 %, preferably at least 98.0 % and more preferably at least 98.5 % of the original pharmaceutical activity of the composition is retained after storage at 40°C and 75 % relative humidity for 3 months.

One preferred pharmaceutical composition of the first, second, third and fourth aspects of the present invention will produce less than 0.2 % by weight, preferably less than 0.1 % by weight and more preferably less than 0.05 % by weight of total impurities from the degradation of the tiotropium bromide monohydrate after storage at 40°C and 75 % relative humidity for 1 month.

Another preferred pharmaceutical composition of the first, second, third and fourth aspects of the present invention will produce less than 0.3 % by weight, preferably less than 0.2 % by weight and more preferably less than 0.15 % by weight of total impurities from the degradation of the tiotropium bromide monohydrate after storage at 40°C and 75 % relative humidity for 3 months.

The weight % of impurities indicated above are based on the total weight of the at least one tiotropium compound and the impurities.

In a further preferred pharmaceutical composition of the first, second, third and fourth aspects of the present invention at least 97.0 % by weight, preferably at least 98.0 % by weight and more preferably at least 98.5 % by weight of the tiotropium bromide monohydrate that is contained originally in the pharmaceutical composition of the invention immediately following preparation will be present in the composition after storage at 40°C and 75 % relative humidity for 3 months.

In yet another preferred pharmaceutical composition of the first, second, third and fourth aspects of the present invention at least 97.0 %, preferably at least 98.0 % and more preferably at least 98.5 % of the original pharmaceutical activity of the pharmaceutical composition of the invention is retained after storage at 40°C and 75 % relative humidity for 3 months.

In referring to the storage of the pharmaceutical compositions in the above described stabilisation methods, we are referring, in particular, to the storage of those compositions in uncoated aluminium containers. Similarly, in referring to the storage of the above described pharmaceutical compositions, we are referring, in particular, to their storage in uncoated aluminium containers.

It has been found that the use of a propellant comprising 1,1-difluoroethane (HFA-152a) in pharmaceutical compositions containing a tiotropium compound, such as tiotropium bromide monohydrate, and the propellant that are designed to be delivered using a metered dose inhaler can unexpectedly improve the aerosolization performance of the pharmaceutical composition after storage when that composition is delivered from the metered dose inhaler compared to the performance that is observed when either HFA-134a or HFA-227ea is used as the propellant. In particular, the fine particle fraction of the tiotropium compound in the emitted dose after storage of the pharmaceutical composition at 50°C and 75 % relative humidity for 15 days is at least 45 weight % of the emitted dose of the tiotropium compound.

Accordingly, in a fifth aspect of the present invention there is provided a method of improving the aerosolization performance after storage of a pharmaceutical composition comprising a propellant component and a drug component comprising tiotropium bromide monohydrate, said method comprising using a propellant component comprising 1,1-difluoroethane (HFA-152a).

The pharmaceutical composition in the method of the fifth aspect of the present invention may be a suspension or a solution.

In a preferred embodiment of the fifth aspect of the present invention there is provided a method of improving the aerosolization performance after storage of a pharmaceutical composition comprising a propellant component and a drug component comprising tiotropium bromide monohydrate, said method comprising using a propellant component comprising 1,1-difluoroethane (HFA-152a) and providing a pharmaceutical composition which when delivered from a metered dose inhaler yields a fine particle fraction of the tiotropium bromide monohydrate which is at least 45 weight % of the emitted dose of the tiotropium bromide monohydrate even after storage of the pharmaceutical composition at 50°C and 75 % relative humidity for 15 days.

Increasing the fine particle fraction of the emitted dose is highly beneficial, because it is the fine drug particles that are able to penetrate into the deep bronchiole passages and the alveolar passages of the lung to maximise relief from the effects of an asthma attack or COPD.

The fine particle fraction is a widely recognised term in the art. It is a measure of the mass fraction of emitted aerosol particles having a diameter below 5 µm which is generally accepted as being the most desirable particle size range for effective alveolar drug delivery.

In the method of the fifth aspect of the present invention, typical and preferred amounts of the drug component and the propellant component and suitable, typical and preferred compositions for the propellant component are as discussed above for the pharmaceutical composition of the first aspect of the invention.

The drug component in the method of the fifth aspect of the present invention may consist essentially of or consist entirely of tiotropium bromide monohydrate. By the term "consists essentially of", we mean that at least 98 weight %, more preferably at least 99 weight % and especially at least 99.9 weight % of the drug component consists of tiotropium bromide monohydrate. Alternatively, the drug component may additionally comprise at least one long acting beta-2 agonist (LABA) and/or at least one corticosteroid. When a long acting beta-2 agonist and/or a corticosteroid are included, suitable and preferred long acting beta-2 agonists and suitable and preferred corticosteroids are as described above for the pharmaceutical compositions of the second and third aspects of the present invention.

In one embodiment, the pharmaceutical composition in the fifth aspect of the present invention consists essentially of and more preferably consists entirely of the drug component and the propellant component as defined above. By the term "consists essentially of", we mean that at least 98 weight %, more preferably at least 99 weight % and especially at least 99.9 weight % of the pharmaceutical composition consists of the two components.

In an alternative embodiment, the pharmaceutical composition in the fifth aspect of the invention may contain one or both of a polar excipient and a surfactant component as discussed above for the pharmaceutical composition of the first aspect of the invention. Suitable and preferred polar excipients and surfactants are as discussed above for the pharmaceutical composition of the first aspect of the invention. Typical and preferred amounts of the polar excipient and the surfactant component are as discussed above for the pharmaceutical composition of the first aspect of the invention.

The pharmaceutical compositions of the invention find particular utility in the delivery of the tiotropium compound, and where included the corticosteroid and long acting beta-2 agonist compounds, from a pressurised aerosol container, e.g. using a metered dose inhaler (MDI). For this application, the pharmaceutical compositions are contained in the pressurised aerosol container and the HFA-152a propellant functions to deliver the drug as a fine aerosol spray.

The pharmaceutical compositions of the invention may comprise one or more other additives of the type that are conventionally used in drug formulations for pressurised MDIs, such as valve lubricants. Where other additives are included in the pharmaceutical compositions, they are normally used in amounts that are conventional in the art.

The pharmaceutical compositions of the invention are normally stored in a pressurised container or canister which is to be used in association with a medication delivery device. When so stored, the pharmaceutical compositions are normally a liquid. In a preferred embodiment, the pressurised container is designed for use in a metered dose inhaler (MDI). In a particularly preferred embodiment, the pressurised container is a coated aluminium can or an uncoated aluminium can, especially the latter.

Accordingly, a sixth aspect of the present invention provides a pressurised container holding the pharmaceutical composition of the first, second, third or fourth aspect of the present invention. In a seventh aspect, the present invention provides a medication delivery device, especially a metered dose inhaler, having a pressurised container holding the pharmaceutical composition of the first, second, third or fourth aspect of the present invention.

The metered dose inhaler typically comprises a nozzle and valve assembly that is crimped to a container holding the pharmaceutical composition to be dispensed. An elastomeric gasket is used to provide a seal between the container and the nozzle/valve assembly. Preferred elastomeric gasket materials are EPDM, chlorobutyl, bromobutyl and cycloolefin copolymer rubbers as these can exhibit good compatibility with HFA-152a and also provide a good barrier to prevent or limit HFA-152a permeating from the container.

The pharmaceutical compositions of the present invention are for use in medicine for treating a patient suffering or likely to suffer from a respiratory disorder and especially asthma or a chronic obstructive pulmonary disease.

Accordingly, also described herein is a method for treating a patient suffering or likely to suffer from a respiratory disorder, especially asthma or a chronic obstructive pulmonary disease, which comprises administering to the patient a therapeutically or prophylactically effective amount of a pharmaceutical composition as discussed above. The pharmaceutical composition is preferably delivered to the patient using a MDI.

The pharmaceutical compositions of the invention can be prepared and the MDI devices filled using techniques that are standard in the art, such as pressure filling and cold filling. For example, the pharmaceutical compositions can be prepared by a simple blending operation in which the at least one tiotropium compound, optionally the at least one corticosteroid and/or the at least one long acting beta-2 agonist, optionally the surfactant component and the HFA-152a-containing propellant are mixed together in the required proportions in a suitable mixing vessel. Mixing can be promoted by stirring as is common in the art. Conveniently, the HFA-152a-containing propellant is liquefied to aid mixing. If the pharmaceutical composition is made in a separate mixing vessel, it can then be transferred to pressurised containers for storage, such as pressurised containers that are used as part of medication delivery devices and especially MDIs.

The pharmaceutical compositions of the invention can also be prepared within the confines of a pressurised container, such as an aerosol canister or vial, from which the compositions are ultimately released as an aerosol spray using a medication delivery device, such as a MDI. In this method, a weighed amount of the at least one tiotropium compound and optionally the at least one corticosteroid and/or at least one long acting beta-2 agonist compound, is introduced into the open container. A valve is then crimped onto the container and the HFA-152a-containing propellant component, in liquid form, introduced through the valve into the container under pressure, optionally after first evacuating the container through the valve. The surfactant component, if included, can be mixed with the drug(s) or, alternatively, introduced into the container after the valve has been fitted, either alone or as a premix with the propellant component. The whole mixture can then be treated to disperse the drugs in the propellant/surfactant mixture, e.g. by vigorous shaking or using an ultrasonic bath. Suitable containers may be made of plastics, metal, e.g. aluminium, or glass. Preferred containers are made of metal, especially aluminium which may be coated or uncoated. Uncoated aluminium containers are especially preferred.

The container may be filled with enough of the pharmaceutical composition to provide for a plurality of dosages. The pressurized aerosol canisters that are used in MDIs typically contain 50 to 150 individual dosages.

Also described is a method of reducing the global warming potential (GWP) of a pharmaceutical composition comprising a drug component comprising at least one tiotropium compound selected from tiotropium and the pharmaceutically acceptable derivatives thereof and a propellant component, said method comprising using a propellant component comprising 1,1-difluoroethane (HFA-152a). This method is applicable to the preparation of all the pharmaceutical compositions disclosed herein in all their aspects and embodiments.

Preferably, at least 90 weight %, more preferably at least 95 weight % and still more preferably at least 99 weight % of the propellant component used is HFA-152a. In an especially preferred method, the propellant component used is entirely HFA-152a.

The propellant component that is used will preferably have a global warming potential (GWP) of less than 250, more preferably less than 200 and still more preferably less than 150.

The present invention is now illustrated but not limited by the following examples.

### Example 1

A number of experiments were conducted to investigate the *in vitro* aerosolization performance of pharmaceutical formulations of tiotropium bromide monohydrate delivered from a metered dose inhaler (MDI) using either HFA-134a or HFA-152a as the propellant.

Pharmaceutical formulations of tiotropium bromide monohydrate were prepared in either HFA-134a or HFA-152a (Mexichem, UK). The drug was weighed directly into standard uncoated 14 ml aluminium canisters (C128, Presspart, Blackburn, UK). The canisters were then crimped with a 50 µL valve (Bespak, Kings Lynn, UK) following which the propellant was filled into the canisters through the valve using a manual Pamasol crimper/filler (Pamasol, Switzerland). Finally, the canisters were sonicated for 20 minutes to aid dispersion of the drug in the suspension. The nominal dose of tiotropium bromide monohydrate was 10µg.

High performance liquid chromatography (HPLC) was used to determine drug content following aerosolization studies (see below). A 150 mm x 3 mm Zorbax SB-C3 propyl-silica column with a 3.5 µm particle size was used for the analysis. The column was coupled to a UV detector operating at a wavelength of 240 nm. The autosampler was operated at ambient temperature and 100 µl samples were injected into the column for the analyses. The chromatographic conditions are shown in Table 1 below.

**Table 1**

| **Drug** | **Pump Flow Rate (ml.min⁻¹)** | **Mobile Phase (gradient elution)** | **UV Wavelength (nm)** | **Column Temperature (°C)** |
|---|---|---|---|---|
| Tiotropium Bromide Monohydrate | 1.20 | Mobile Phase A: Sodium methane sulphonate/potassium dihydrogen phosphate | 240 | 50 |
| | | Mobile Phase B: Methanol/Acetonitrile (10:40 v/v) | | |

The *in vitro* aerosolization performance of the formulations was studied using a Next Generation Impactor (NGI, Copley Scientific, Nottingham UK), which was connected to a vacuum pump (GE Motors, NJ, USA). Prior to testing, the cups of the NGI system were coated with 1 % v/v silicone oil in hexane to eliminate particle bounce. For each experiment, three actuations of the valve were discharged into the NGI at 30 L.min⁻¹ as per pharmacopeia guidelines. Following aerosolization, the NGI apparatus was dismantled and the actuator and each part of the NGI was washed down into known volumes of the HPLC mobile phase. The mass of drug deposited on each part of the NGI was determined by HPLC using the methodology described above. This protocol was repeated three times for each canister, following which, the fine particle dose (FPD) and fine particle fraction of the emitted dose (FPF_{ED}) were determined. The results are shown in Table 2 below.

**Table 2. In vitro aerosolization performance of tiotropium bromide monohydrate in HFA-134a and HFA-152a as characterised by the emitted dose, fine particle dose, fine particle fraction of the emitted dose (FPF_{ED} %), mass median aerodynamic diameter (MMAD) and geometric standard deviation (GSD).**

| | HFA-134a | HFA-152a |
|---|---|---|
| Emitted Dose (µg ± S.D.) | 7.5 ± 0.1 | 7.2 ± 0.2 |
| Fine Particle Dose (µg ± S.D.) | 2.4 ± 0.2 | 2.7 ± 0.1 |
| FPF_{ED} % ± S.D. | 31.4 ± 2.5 | 38.0 ± 0.8 |
| MMAD (µm) | 4.8 | 4.5 |
| GSD | 2.1 | 2.1 |

### Example 2

A number of experiments were conducted to investigate the *in vitro* aerosolization performance of pharmaceutical formulations of tiotropium bromide monohydrate delivered from a metered dose inhaler (MDI) using either HFA-134a, HFA-227ea or HFA-152a as the propellant after initial preparation and after storing under stress storage conditions. The experimental protocol described above was used to prepare the pharmaceutical formulations and the *in vitro* aerosolization performance of the formulations was tested immediately after preparation (time t = zero) with a Next Generation Impactor using the method described in Example 1 above. The formulations were then stored under stress storage conditions (valve down) at 50°C and 75 % relative humidity for 5 days and 15 days. After storing for 5 days and 15 days under the stress storage conditions, the *in vitro* aerosolization performance of the pharmaceutical formulations was tested again as before with a Next Generation Impactor using the method described in Example 1 above. The results are shown in Tables 3 to 5 below.

**Table 3. In vitro aerosolization performance of tiotropium bromide monohydrate delivered from a MDI using HFA-227ea, HFA-134a or HFA-152a as the propellant at time t = zero as characterised by the fine particle dose, fine particle fraction of the emitted dose (FPF_{ED} %), mass median aerodynamic diameter (MMAD) and geometric standard deviation (GSD).**

| | HFA-227ea T=0 | HFA-134a T=0 | HFA-152a T=0 |
|---|---|---|---|
| Fine Particle Dose (µg) | 2.26 | 5.67 | 2.70 |
| FPF_{ED} % | 41.07 | 47.22 | 44.12 |
| MMAD (µm) | 3.12 | 2.68 | 2.59 |
| GSD | 1.84 | 1.68 | 1.65 |

**Table 4. In vitro aerosolization performance of tiotropium bromide monohydrate delivered from a MDI using HFA-227ea, HFA-134a or HFA-152a as the propellant after storage (valve down) for 5 days at 50°C and 75 % relative humidity as characterised by the fine particle dose, fine particle fraction of the emitted dose (FPF_{ED} %), mass median aerodynamic diameter (MMAD) and geometric standard deviation (GSD).**

| | HFA-227ea T=5 days @ 50°C and 75% RH | HFA-134a T=5 days @ 50°C and 75% RH | HFA-152a T=5 days @ 50°C and 75% RH |
|---|---|---|---|
| Fine Particle Dose (µg) | 0.99 | 2.59 | 4.12 |
| FPF_{ED} % | 13.77 | 31.82 | 47.47 |
| MMAD (µm) | 8.57 | 2.07 | 2.73 |
| GSD | 2.06 | 1.80 | 1.72 |

**Table 5. In vitro aerosolization performance of tiotropium bromide monohydrate delivered from a MDI using HFA-227ea, HFA-134a or HFA-152a as the propellant after storage (valve down) for 15 days at 50°C and 75 % relative humidity as characterised by the fine particle dose, fine particle fraction of the emitted dose (FPF_{ED} %), mass median aerodynamic diameter (MMAD) and geometric standard deviation (GSD).**

| | HFA-227ea T=15 days @ 50°C and 75% RH | HFA-134a T=15 days @ 50°C and 75% RH | HFA-152a T=15 days @ 50°C and 75% RH |
|---|---|---|---|
| Fine Particle Dose (µg) | 0.79 | 3.72 | 5.51 |
| FPF_{ED} % | 13.02 | 41.10 | 50.27 |
| MMAD (µm) | 8.54 | 2.12 | 2.81 |
| GSD | 1.96 | 1.67 | 1.73 |

When HFA-227ea was used as the propellant to aerosolize the tiotropium bromide monohydrate, the aerosolization performance decreased dramatically after the pharmaceutical formulation containing the drug and the propellant had been stored under stress storage conditions for 5 days and 15 days at 50°C and 75% relative humidity. In particular, the fine particle dose and fine particle fraction of the emitted dose decreased dramatically.

When HFA-134a was used as the propellant to aerosolize the tiotropium bromide monohydrate, the aerosolization performance decreased significantly after the pharmaceutical formulation containing the drug and the propellant had been stored under stress storage conditions for 5 days and 15 days at 50°C and 75% relative humidity. In particular, the fine particle dose and fine particle fraction of the emitted dose decreased appreciably.

In contrast, when HFA-152a was used as the propellant to aerosolize the tiotropium bromide monohydrate, a good aerosolization performance was maintained after the pharmaceutical formulation containing the drug and the propellant had been stored under stress storage conditions for 5 days and 15 days at 50°C and 75% relative humidity.

### Example 3

The chemical stability of tiotropium bromide monohydrate in HFA-134a and HFA-152a was investigated at time zero (T=0) and after storage, valve down, for 1 month (T=1M) and 3 months (T=3M) at 40°C and 75% relative humidity (RH) and at 25°C and 60% relative humidity (RH) in uncoated aluminium cans.

The drug formulations were prepared as described in Example 1 above and analysed using high performance liquid chromatography (HPLC). A 150 mm x 4.6 mm Accucore C18 column with a 2.6 µm particle size was used for the analysis. The column was coupled to a UV detector operating at a wavelength of 240 nm. The autosampler was operated at ambient temperature and 100 µl samples were injected into the column for the analyses. The chromatographic conditions are shown in Table 6 below.

**Table 6**

| **Pump Flow Rate (ml.min⁻¹)** | **Mobile Phase (gradient elution)** | **UV Wavelength (nm)** | **Column Temperature (°C)** |
|---|---|---|---|
| 1.0 | Mobile Phase A: 10mM Ammonium formate (pH 3.0) | 240 | 45 |
| | Mobile Phase B: Acetonitrile | | |

The composition of the mobile phase was varied as shown in Table 7 below.

**Table 7**

| Time (minutes) | % Mobile phase A | % Mobile phase B |
|---|---|---|
| 0 | 95 | 5 |
| 1 | 95 | 5 |
| 21 | 0 | 100 |
| 22 | 0 | 100 |
| 23 | 95 | 5 |
| 28 | 95 | 5 |

The results of investigating the chemical stability of the tiotropium bromide monohydrate drug formulations in HFA-152a and HFA-134a in uncoated aluminium cans are shown, respectively, in Tables 8 and 9 below.

**Table 8. Chemical stability of tiotropium bromide monohydrate in HFA-134a in uncoated aluminium cans based on percentage assay and total impurities upon storage at T=0, T=1M @ 40°C/75 % RH and 25°C/60 % RH and T=3M @ 40°C/75 % RH and 25°C/60 % RH.**

| Time | % Assay (LC) | % total impurities |
|---|---|---|
| Initial time T = 0 | 99.8 | 0.08 |
| T = 1M @ 25/60 | 99.8 | 0.13 |
| T = 1M @ 40/75 | 99.5 | 0.28 |
| T = 3M @ 25/60 | 97.8 | 0.32 |
| T = 3M @ 40/75 | 96.4 | 0.44 |

**Table 9. Chemical stability of tiotropium bromide monohydrate (TBM) in HFA-152a in uncoated aluminium cans based on percentage assay and total impurities upon storage at T=0, T=1M @ 40°C/75 % RH and 25°C/60 % RH and T=3M @ 40°C/75 % RH and 25°C/60 % RH.**

| Time | % Assay (LC) | % total impurities |
|---|---|---|
| Initial time T = 0 | 100.5 | <LoQ |
| T = 1M @ 25/60 | 99.9 | <LoQ |
| T = 1M @ 40/75 | 99.8 | <LoQ |
| T = 3M @ 25/60 | 98.9 | 0.08 |
| T = 3M @ 40/75 | 98.5 | 0.13 |

It can be seen from the above data that pharmaceutical formulations of tiotropium bromide monohydrate exhibit superior chemical stability when blended together with HFA-152a as the aerosolization propellant.

### Example 4

Formulations containing tiotropium bromide monohydrate and either HFA-134a or HFA-152a were prepared in PET vials and the suspension stability of the formulations determined using a Turbiscan MA 2000. The Turbiscan instrument has a reading head that moves along a flat-bottomed, 5 mL cylindrical glass cell, and takes readings of transmitted and backscattered light every 40 µm on a maximum sample height of 80 mm. The reading head uses a pulsed near infrared light source and two synchronous detectors. The transmission detector picks up light transmitted through the suspension tube at 0° and back scattering detector receives light back by the product at 135°.

The sedimentation and size of flocs for the different formulations are shown in Table 10 below.

**Table 10. Suspension stability profiles of tiotropium bromide monohydrate formulations in HFA-134a and HFA-152a.**

| **Formulation** | **Size Start (microns)** | **Time to sediment (mins)** |
|---|---|---|
| Tiotropium bromide monohydrate and HFA-134a | 3.45 | 0.82 |
| Tiotropium bromide monohydrate and HFA-152a | 3.55 | 0.91 |

## Claims

1. A pharmaceutical composition comprising:
(i) a drug component comprising tiotropium bromide monohydrate; and
(ii) a propellant component at least 90 weight % of which is 1,1-difluoroethane (HFA-152a),
wherein the composition is a solution and is free of acid stabilisers.

2. A pharmaceutical composition comprising:
(i) a drug component comprising tiotropium bromide monohydrate; and
(ii) a propellant component at least 90 weight % of which is 1,1-difluoroethane (HFA-152a),
wherein the composition is a suspension.

3. The pharmaceutical composition of claim 2, wherein the composition is free of acid stabilisers.

4. The pharmaceutical composition of any one of claims 1 to 3, wherein the composition contains less than 500 ppm, preferably less than 100 ppm, more preferably less than 50 ppm, still more preferably less than 10 ppm and especially less than 5 ppm of water based on the total weight of the pharmaceutical composition.

5. The pharmaceutical composition of claim 4, wherein the composition contains greater than 0.5 ppm, e.g. greater than 1 ppm, of water based on the total weight of the pharmaceutical composition.

6. The pharmaceutical composition of any one of the preceding claims, wherein the composition contains less than 1000 ppm, preferably less than 500 ppm, more preferably less than 100 ppm and particularly less than 50 ppm of oxygen based on the total weight of the pharmaceutical composition.

7. The pharmaceutical composition of claim 6, wherein the composition contains greater than 0.5 ppm, e.g. greater than 1 ppm, of oxygen based on the total weight of the pharmaceutical composition.

8. The pharmaceutical composition of any one of the preceding claims, wherein the drug component additionally comprises at least one long acting beta-2-agonist (LABA).

9. The pharmaceutical composition of claim 8, wherein the at least one long acting beta-2-agonist is selected from the group consisting of formoterol, salmeterol and the pharmaceutically acceptable salts thereof, preferably from the group consisting of formoterol fumarate, formoterol fumarate dihydrate and salmeterol xinafoate.

10. The pharmaceutical composition of any one of the preceding claims, wherein the drug component additionally comprises at least one corticosteroid.

11. The pharmaceutical composition of claim 10, wherein the at least one corticosteroid is selected from mometasone, beclomethasone, fluticasone and the pharmaceutically acceptable salts and esters thereof, preferably from the group consisting of mometasone, mometasone furoate, beclomethasone, beclomethasone dipropionate, fluticasone and fluticasone propionate.

12. The pharmaceutical composition of any one of the preceding claims, wherein at least 95 weight % and preferably at least 99 weight % of the propellant component is 1,1-difluoroethane (HFA-152a).

13. The pharmaceutical composition of any one of claims 1 to 11, wherein the propellant component is entirely 1,1-difluoroethane (HFA-152a).

14. The pharmaceutical composition of claim 12 or claim 13, wherein the propellant component contains from 0.5 to 10 ppm, e.g. from 1 to 5 ppm, of unsaturated impurities.

15. The pharmaceutical composition of any one of the preceding claims further comprising a surfactant component comprising at least one surfactant compound, preferably at least one surfactant compound selected from polyvinylpyrrolidone, polyethylene glycol surfactants, oleic acid and lecithin.

16. The pharmaceutical composition of any one of the preceding claims further comprising a polar excipient, preferably ethanol.

17. The pharmaceutical composition of any one of claims 1 to 15, wherein the pharmaceutical composition is free of one or more of the following: (i) perforated microstructures; (ii) pharmaceutically acceptable salts of both cromoglycic acid and nedocromil; (iii) polar excipients; and (iv) ethanol.

18. The pharmaceutical composition of any one of the preceding claims which after storage in uncoated aluminium containers at 40°C and 75 % relative humidity for 1 month will produce less than 0.2 % by weight, preferably less than 0.1 % by weight and more preferably less than 0.05 % by weight of impurities from the degradation of the tiotropium bromide monohydrate based on the total weight of the tiotropium bromide monohydrate and the impurities.

19. The pharmaceutical composition of any one of the preceding claims which after storage in uncoated aluminium containers at 40°C and 75 % relative humidity for 3 months will produce less than 0.3 % by weight, preferably less than 0.2 % by weight and more preferably less than 0.15 % by weight of impurities from the degradation of the tiotropium bromide monohydrate based on the total weight of the tiotropium bromide monohydrate and the impurities.

20. The pharmaceutical composition of any one of the preceding claims, wherein at least 97.0 % by weight, preferably at least 98.0 % by weight and more preferably at least 98.5 % by weight of the tiotropium bromide monohydrate that is contained originally in the pharmaceutical composition immediately following preparation will be present in the composition after storage in uncoated aluminium containers at 40°C and 75 % relative humidity for 3 months.

21. A metered dose inhaler (MDI) fitted with a sealed and pressurised aerosol container containing a pharmaceutical composition as claimed in any one of claims 1 to 20.

22. A method of improving the aerosolization performance after storage of a pharmaceutical composition comprising a propellant component and a drug component comprising tiotropium bromide monohydrate, said method comprising using a propellant component at least 90 weight % of which is 1,1-difluoroethane (HFA-152a).

23. The method of claim 22, wherein the method provides a pharmaceutical composition which when delivered from a metered dose inhaler yields a fine particle fraction of the tiotropium bromide monohydrate which is at least 45 weight % of the emitted dose of the tiotropium bromide monohydrate even after storage of the pharmaceutical composition at 50°C and 75 % relative humidity for 15 days.

24. The method of claim 22 or 23, wherein the aerosolization performance of the pharmaceutical composition is improved compared to a pharmaceutical composition that uses 1,1,1,2-tetrafluoroethane (HFA-134a) or 1,1,1,2,3,3,3-heptafluoropropane (HFA-227ea) as the propellant.

25. The method of any one of claims 22 to 24, wherein the pharmaceutical composition is a composition as claimed in any one of claims 1 to 20.

26. The pharmaceutical composition of any one of claims 1 to 20 which when delivered from a metered dose inhaler yields a fine particle fraction of the tiotropium bromide monohydrate which is at least 45 weight % of the emitted dose of the tiotropium bromide monohydrate even after storage of the pharmaceutical composition at 50°C and 75 % relative humidity for 15 days.

27. The pharmaceutical composition of any one of claims 8 to 11 which is adapted to deliver the compounds making up the drug component in approximately the same proportions that they occur in the pharmaceutical composition.

## Patentansprüche

1. Pharmazeutische Zusammensetzung, die Folgendes umfasst:
(i) eine Arzneimittelkomponente, die Tiotropiumbromidmonohydrat umfasst; und
(ii) eine Treibmittelkomponente, von der wenigstens 90 Gew.-% 1,1-Difluorethan (HFA-152a) sind,
wobei die Zusammensetzung eine Lösung ist und frei von Säurestabilisatoren ist.

2. Pharmazeutische Zusammensetzung, die Folgendes umfasst:
(i) eine Arzneimittelkomponente, die Tiotropiumbromidmonohydrat umfasst; und
(ii) eine Treibmittelkomponente, von der wenigstens 90 Gew.-% 1,1-Difluorethan (HFA-152a) sind,
wobei die Zusammensetzung eine Suspension ist.

3. Pharmazeutische Zusammensetzung nach Anspruch 2, wobei die Zusammensetzung frei von Säurestabilisatoren ist.

4. Pharmazeutische Zusammensetzung nach einem der Ansprüche 1 bis 3, wobei die Zusammensetzung weniger als 500 ppm, bevorzugt weniger als 100 ppm, stärker bevorzugt weniger als 50 ppm, noch stärker bevorzugt weniger als 10 ppm und besonders weniger als 5 ppm Wasser basierend auf dem Gesamtgewicht der pharmazeutischen Zusammensetzung enthält.

5. Pharmazeutische Zusammensetzung nach Anspruch 4, wobei die Zusammensetzung über 0,5 ppm, z. B. über 1 ppm, Wasser basierend auf dem Gesamtgewicht der pharmazeutischen Zusammensetzung enthält.

6. Pharmazeutische Zusammensetzung nach einem der vorhergehenden Ansprüche, wobei die Zusammensetzung weniger als 1000 ppm, bevorzugt weniger als 500 ppm, stärker bevorzugt weniger als 100 ppm und insbesondere weniger als 50 ppm Sauerstoff basierend auf dem Gesamtgewicht der pharmazeutischen Zusammensetzung enthält.

7. Pharmazeutische Zusammensetzung nach Anspruch 6, wobei die Zusammensetzung über 0,5 ppm, z. B. über 1 ppm, Sauerstoff basierend auf dem Gesamtgewicht der pharmazeutischen Zusammensetzung enthält.

8. Pharmazeutische Zusammensetzung nach einem der vorhergehenden Ansprüche, wobei die Arzneimittelkomponente zusätzlich wenigstens einen lang wirkenden Beta-2-Agonisten (long acting beta agonist - LABA) umfasst.

9. Pharmazeutische Zusammensetzung nach Anspruch 8, wobei der wenigstens eine lang wirkende Beta-2-Agonist aus der Gruppe, die aus Formoterol, Salmeterol und den pharmazeutisch unbedenklichen Salzen davon besteht, ausgewählt ist, bevorzugt aus der Gruppe, die aus Formoterolfumarat, Formoterolfumaratdihydrat und Salmeterolxinafoat besteht.

10. Pharmazeutische Zusammensetzung nach einem der vorhergehenden Ansprüche, wobei die Arzneimittelkomponente zusätzlich wenigstens ein lang wirkendes Corticosteroid umfasst.

11. Pharmazeutische Zusammensetzung nach Anspruch 10, wobei das wenigstens eine Corticosteroid aus Mometason, Beclomethason, Fluticason und den pharmazeutisch unbedenklichen Salzen und Estern davon ausgewählt ist, bevorzugt aus der Gruppe, die aus Mometason, Mometasonfuroat, Beclomethason, Beclomethasondipropionat, Fluticason und Fluticasonpropionat besteht.

12. Pharmazeutische Zusammensetzung nach einem der vorhergehenden Ansprüche, wobei wenigstens 95 Gew.-% und bevorzugt wenigstens 99 Gew.-% der Treibmittelkomponente 1,1-Difluorethan (HFA-152a) sind.

13. Pharmazeutische Zusammensetzung nach einem der Ansprüche 1 bis 11, wobei die Treibmittelkomponente gänzlich 1,1-Difluorethan (HFA-152a) ist.

14. Pharmazeutische Zusammensetzung nach Anspruch 12 oder 13, wobei die Treibmittelkomponente von 0,5 bis 10 ppm, z. B. von 1 bis 5 ppm, ungesättigte Verunreinigungen enthält.

15. Pharmazeutische Zusammensetzung nach einem der vorhergehenden Ansprüche, die ferner eine Tensidkomponente umfasst, die wenigstens eine Tensidverbindung, bevorzugt wenigstens eine Tensidverbindung, die aus Polyvinylpyrrolidon, Polyethylenglycoltensiden, Ölsäure und Lecithin ausgewählt ist, umfasst.

16. Pharmazeutische Zusammensetzung nach einem der vorhergehenden Ansprüche, die ferner einen polaren Arzneistoffträger, bevorzugt Ethanol, umfasst.

17. Pharmazeutische Zusammensetzung nach einem der Ansprüche 1 bis 15, wobei die pharmazeutische Zusammensetzung frei von Folgendem ist: (i) perforierten Mikrostrukturen; (ii) pharmazeutisch unbedenklichen Salzen sowohl von Cromoglicinsäure als auch von Nedocromil; (iii) polaren Arzneistoffträgern; und/oder (iv) Ethanol.

18. Pharmazeutische Zusammensetzung nach einem der vorhergehenden Ansprüche, die nach Lagerung in unbeschichteten Aluminiumbehältern bei 40 °C und 75 % relativer Feuchtigkeit 1 Monat lang weniger als 0,2 Gew.-%, bevorzugt weniger als 0,1 Gew.-% und stärker bevorzugt weniger als 0,05 Gew.-%, Verunreinigungen aus dem Abbau des Tiotropiumbromidmonohydrats, basierend auf dem Gesamtgewicht des Tiotropiumbromidmonohydrats und den Verunreinigungen, erzeugen wird.

19. Pharmazeutische Zusammensetzung nach einem der vorhergehenden Ansprüche, die nach Lagerung in unbeschichteten Aluminiumbehältern bei 40 °C und 75 % relativer Feuchtigkeit 3 Monate lang weniger als 0,3 Gew.-%, bevorzugt weniger als 0,2 Gew.-% und stärker bevorzugt weniger als 0,15 Gew.-%, Verunreinigungen aus dem Abbau des Tiotropiumbromidmonohydrats, basierend auf dem Gesamtgewicht des Tiotropiumbromidmonohydrats und den Verunreinigungen, erzeugen wird.

20. Pharmazeutische Zusammensetzung nach einem der vorhergehenden Ansprüche, wobei wenigstens 97,0 Gew.-%, bevorzugt wenigstens 98,0 Gew.-% und stärker bevorzugt wenigstens 98,5 Gew.-% des Tiotropiumbromidmonohydrats, das in der pharmazeutischen Zusammensetzung sofort anschließend an eine Herstellung ursprünglich enthalten ist, in der Zusammensetzung nach Lagerung in unbeschichteten Aluminiumbehältern bei 40 °C und 75 % relativer Feuchtigkeit 3 Monate lang vorhanden sein wird.

21. Dosierinhalator (metered dose inhaler - MDI), der mit einem versiegelten und unter Druck stehenden Aerosolbehälter, der eine pharmazeutische Zusammensetzung nach einem der Ansprüche 1 bis 20 enthält, ausgestattet ist.

22. Verfahren zum Verbessern der Aerosolisierungsleistung nach Lagerung einer pharmazeutischen Zusammensetzung, die eine Treibmittelkomponente und eine Arzneimittelkomponente umfasst, die Tiotropiumbromidmonohydrat umfasst, wobei das Verfahren ein Verwenden einer Treibmittelkomponente umfasst, von der wenigstens 90 Gew.-% 1,1-Difluorethan (HFA-152a) sind.

23. Verfahren nach Anspruch 22, wobei das Verfahren eine pharmazeutische Zusammensetzung bereitstellt, die, wenn sie von einem Dosierinhalator abgegeben wird, eine Feinpartikelfraktion des Tiotropiumbromidmonohydrats ergibt, die wenigstens 45 Gew.-% der emittierten Dosis des Tiotropiumbromidmonohydrats beträgt, selbst nach Lagerung der pharmazeutischen Zusammensetzung bei 50 °C und 75 % relativer Feuchtigkeit 15 Tage lang.

24. Verfahren nach Anspruch 22 oder 23, wobei die Aerosolisierungsleistung der pharmazeutischen Zusammensetzung verglichen mit einer pharmazeutischen Zusammensetzung, die 1,1,1,2-Tetrafluorethan (HFA-134a) oder 1,1,1,2,3,3,3-Heptafluorpropan (HFA-227ea) als das Treibmittel verwendet, verbessert ist.

25. Verfahren nach einem der Ansprüche 22 bis 24, wobei die pharmazeutische Zusammensetzung eine Zusammensetzung nach einem der Ansprüche 1 bis 20 ist.

26. Pharmazeutische Zusammensetzung nach einem der Ansprüche 1 bis 20, die, wenn sie von einem Dosierinhalator abgegeben wird, eine Feinpartikelfraktion des Tiotropiumbromidmonohydrats ergibt, die wenigstens 45 Gew.-% der emittierten Dosis des Tiotropiumbromidmonohydrats beträgt, selbst nach Lagerung der pharmazeutischen Zusammensetzung bei 50 °C und 75 % relativer Feuchtigkeit 15 Tage lang.

27. Pharmazeutische Zusammensetzung nach einem der Ansprüche 8 bis 11, die angepasst ist, um die Verbindungen, die die Arzneimittelkomponente ausmachen, in ungefähr den gleichen Anteilen abzugeben, in denen sie in der pharmazeutischen Zusammensetzung auftreten.

## Revendications

1. Composition pharmaceutique comprenant :
(i) un constituant médicamenteux comprenant du bromure de tiotropium monohydraté ; et
(ii) un constituant propulseur dont au moins 90 % en poids est du 1,1-difluoroéthane (HFA-152a),
la composition étant une solution et étant exempte de stabilisants acides.

2. Composition pharmaceutique comprenant :
(i) un constituant médicamenteux comprenant du bromure de tiotropium monohydraté ; et
(ii) un constituant propulseur dont au moins 90 % en poids est du 1,1-difluoroéthane (HFA-152a),
la composition étant une suspension.

3. Composition pharmaceutique selon la revendication 2, la composition étant exempte de stabilisants acides.

4. Composition pharmaceutique selon l'une quelconque des revendications 1 à 3, la composition contenant moins de 500 ppm, de préférence moins de 100 ppm, plus préférablement moins de 50 ppm, encore plus préférablement moins de 10 ppm et en particulier moins de 5 ppm d'eau sur la base du poids total de la composition pharmaceutique.

5. Composition pharmaceutique selon la revendication 4, la composition contenant plus de 0,5 ppm, par exemple plus de 1 ppm, d'eau sur la base du poids total de la composition pharmaceutique.

6. Composition pharmaceutique selon l'une quelconque des revendications précédentes, la composition contenant moins de 1 000 ppm, de préférence moins de 500 ppm, plus préférablement moins de 100 ppm et en particulier moins de 50 ppm d'oxygène sur la base du poids total de la composition pharmaceutique.

7. Composition pharmaceutique selon la revendication 6, la composition contenant plus de 0,5 ppm, par exemple plus de 1 ppm, d'oxygène sur la base du poids total de la composition pharmaceutique.

8. Composition pharmaceutique selon l'une quelconque des revendications précédentes, le constituant médicamenteux comprenant en outre au moins un bêta-2 agoniste à longue durée d'action (BALA).

9. Composition pharmaceutique selon la revendication 8, l'au moins un bêta-2-agoniste à longue durée d'action étant choisi dans le groupe constitué de formotérol, salmétérol et leurs sels pharmaceutiquement acceptables, de préférence dans le groupe constitué de fumarate de formotérol, fumarate de formotérol dihydraté et xinafoate de salmétérol.

10. Composition pharmaceutique selon l'une quelconque des revendications précédentes, le constituant médicamenteux comprenant d'autre part au moins un corticostéroïde.

11. Composition pharmaceutique selon la revendication 10, l'au moins un corticostéroïde étant choisi parmi la mométasone, la béclométhasone, la fluticasone et leurs sels et esters pharmaceutiquement acceptables, de préférence parmi le groupe constitué de mométasone, furoate de mométasone, béclométhasone, dipropionate de béclométhasone, fluticasone et propionate de fluticasone.

12. Composition pharmaceutique selon l'une quelconque des revendications précédentes, au moins 95 % en poids et de préférence au moins 99 % en poids du constituant propulseur étant du 1,1-difluoroéthane (HFA-152a).

13. Composition pharmaceutique selon l'une quelconque des revendications 1 à 11, l'ensemble du constituant propulseur étant du 1,1-difluoroéthane (HFA-152a).

14. Composition pharmaceutique selon l'une quelconque des revendications 12 ou 13, le constituant propulseur contenant de 0,5 à 10 ppm, par exemple de 1 à 5 ppm, d'impuretés insaturées.

15. Composition pharmaceutique selon l'une quelconque des revendications précédentes comprenant en outre un constituant surfactant comprenant au moins un composé surfactant, de préférence au moins un composé surfactant choisi parmi la polyvinylpyrrolidone, les surfactants de polyéthylèneglycol, l'acide oléique et la lécithine.

16. Composition pharmaceutique selon l'une quelconque des revendications précédentes, comprenant en outre un excipient polaire, de préférence de l'éthanol.

17. Composition pharmaceutique selon l'une quelconque des revendications 1 à 15, la composition pharmaceutique étant exempte : (i) de microstructures perforées ; et/ou (ii) de sels pharmaceutiquement acceptables à la fois d'acide cromoglycique et de nédocromil ; et/ou (iii) d'excipients polaires ; et/ou (iv) d'éthanol.

18. Composition pharmaceutique selon l'une quelconque des revendications précédentes, laquelle, après avoir été stockée dans des récipients en aluminium non revêtus à 40 °C et à 75 % d'humidité relative pendant 1 mois, produit moins de 0,2 % en poids, de préférence moins de 0,1 % en poids, et plus préférablement moins de 0,05 % en poids d'impuretés provenant de la dégradation du bromure de tiotropium monohydraté sur la base du poids total du bromure de tiotropium monohydraté et des impuretés.

19. Composition pharmaceutique selon l'une quelconque des revendications précédentes, laquelle, après avoir été stockée dans des récipients en aluminium non revêtus à 40 °C et à 75 % d'humidité relative pendant 3 mois, produit moins de 0,3 % en poids, de préférence moins de 0,2 % en poids, et plus préférablement moins de 0,15 % en poids d'impuretés provenant de la dégradation du bromure de tiotropium monohydraté sur la base du poids total du bromure de tiotropium monohydraté et des impuretés.

20. Composition pharmaceutique selon l'une quelconque des revendications précédentes, au moins 97,0 % en poids, de préférence au moins 98,0 % en poids et plus préférablement au moins 98,5 % en poids du bromure de tiotropium monohydraté qui est contenu à l'origine dans la composition pharmaceutique immédiatement après sa préparation étant présent dans la composition après stockage dans des récipients en aluminium non revêtus à 40 °C et à 75 % d'humidité relative pendant 3 mois.

21. Aérosol-doseur (MDI) équipé d'un récipient aérosol étanche et sous pression contenant une composition pharmaceutique selon l'une quelconque des revendications 1 à 20.

22. Procédé d'amélioration de la performance d'aérosolisation d'une composition pharmaceutique comprenant un constituant propulseur et un constituant médicamenteux comprenant du bromure de tiotropium monohydraté, ledit procédé comprenant l'utilisation d'un constituant propulseur dont au moins 90 % en poids est du 1,1-difluoroéthane (HFA-152a).

23. Procédé selon la revendication 22, le procédé fournissant une composition pharmaceutique qui, lorsqu'elle est administrée à partir d'un aérosol-doseur, donne une fraction de particules fines du bromure de tiotropium monohydraté qui est au moins 45 % en poids de la dose émise du bromure de tiotropium monohydraté même après le stockage de la composition pharmaceutique à 50 °C et 75 % d'humidité relative pendant 15 jours.

24. Procédé selon l'une quelconque des revendications 22 ou 23, les performances d'aérosolisation de la composition pharmaceutique étant améliorées par rapport à une composition pharmaceutique qui utilise du 1,1,1,2-tétrafluoroéthane (HFA-134a) ou du 1,1,1,2,3,3,3-heptafluoropropane (HFA-227ea) comme propulseur.

25. Procédé selon l'une quelconque des revendications 22 à 24, la composition pharmaceutique étant une composition selon l'une quelconque des revendications 1 à 20.

26. Composition pharmaceutique selon l'une quelconque des revendications 1 à 20 qui, lorsqu'elle est administrée à partir d'un aérosol-doseur, donne une fraction de particules fines du bromure de tiotropium monohydraté qui est au moins 45 % en poids de la dose émise du bromure de tiotropium monohydraté même après stockage de la composition pharmaceutique à 50 °C et 75 % d'humidité relative pendant 15 jours.

27. Composition pharmaceutique selon l'une quelconque des revendications 8 à 11, qui est apte à administrer les composés formant le constituant médicamenteux à raison de proportions approximativement identiques à celles à raison desquelles ils apparaissent dans la composition pharmaceutique.
